Europäisches Patentamt

European Patent Office

Office européen des brevets

(11) Veröffentlichungsnummer: **0 219 045**
**A2**

(12) **EUROPÄISCHE PATENTANMELDUNG**

(21) Anmeldenummer: **86113880.8**

(22) Anmeldetag: **07.10.86**

(51) Int. Cl.⁴: **C07C 47/127** , **C07C 45/79**

(30) Priorität: **11.10.85 DE 3536263**

(43) Veröffentlichungstag der Anmeldung:
**22.04.87 Patentblatt 87/17**

(84) Benannte Vertragsstaaten:
**BE DE FR GB IT NL**

(71) Anmelder: **BASF Aktiengesellschaft**
**Carl-Bosch-Strasse 38**
**D-6700 Ludwigshafen(DE)**

(72) Erfinder: **Mueller, Guenther, Dr.**
**Im Zinkig 92**
**D-6700 Ludwigshafen(DE)**
Erfinder: **Ramsteiner, Rolf**
**Am Schlittweg 13**
**D-6708 Neuhofen(DE)**
Erfinder: **Graf, Fritz, Dr.**
**Im Rothschild 33**
**D-6720 Speyer(DE)**
Erfinder: **Hupfer, Leopold, Dr.**
**Waltershoehe 3**
**D-6701 Friedelsheim(DE)**

(54) **Verfahren zur Entfärbung wässriger Glyoxallösungen.**

(57) Verfahren zur Entfärbung wäßriger Glyoxallösungen, bei dem man die Glyoxallösung über eine im Festbett angeordnete granulierte Aktivkohle leitet, deren differentielle Porenradienverteilung ein Maximum von 10 bis 100 A aufweist, und wobei die Peclet-Zahl zwischen 500 und 5000 liegt, das Aktivkohlebett nach dem Ablauf der entfärbten Glyoxallösung zur Regenerierung mit einer wäßrigen Lösung eines alkalisch wirkenden Mittels und danach mit einer wäßrigen Säure behandelt, und über die so regenerierte Aktivkohle erneut zu entfärbende Glyoxallösung leitet.

EP 0 219 045 A2

## Verfahren zur Entfärbung wäßriger Glyoxallösungen

Glyoxal wird als etwa 40 gew.%ige wäßrige Lösung auf den Markt gebracht. Von diesen Glyoxallösungen, die z.B. für die Herstellung von Textil- und Papierhilfsmitteln oder für die Zubereitung von Desinfektionsmitteln verwendet werden, wird verlangt, daß sie eine Farbzahl von ≤10 APHA aufweisen.

Technisch stellt man Glyoxal durch Oxidation von Acetaldehyd mit Salpetersäure oder durch oxidative Dehydrierung von Ethylenglykol an speziellen Katalysatoren her. In beiden Fällen werden wäßrige Glyoxal-Rohlösungen erhalten, die als Nebenprodukte Säuren enthalten und mehr oder weniger stark gelb gefärbt sind.

Es hat deshalb nicht an Versuchen gefehlt, diese rohen Glyoxallösungen zu reinigen und zu entfärben. Nach den Angaben der US-PS 3 270 062 oder der DE-OS 3 402 733 entfernt man Nebenprodukte, wie Essigsäure, Ameisensäure und Glykolsäure durch eine Behandlung der Rohlösungen mit fest angeordneten Anionenaustauschern oder mit Lösungen tertiärer Amine. Bei diesen Verfahren wird auch ein Teil der für die Gelbfärbung verantwortlichen chemisch nicht näher identifizierten Komponenten zurückgehalten. Da jedoch die Gelbfärbung bereits weit vor Erschöpfung des Säureaufnahmevermögens der Anionenaustauscher durchbricht, gelingt es nicht, auf wirtschaftliche Art und Weise, Glyoxal-Lösungen mit einer Farbzahl ≤10 APHA herzustellen. Üblicherweise schließt man deshalb an die Säureentfernung eine Entfärbung der Glyoxal-Lösung mit pulverisierter Aktivkohle an, welche durch Umwälzen der Lösung oder durch intensives Rühren mechanisch in Schwebe gehalten und nach einer Einwirkungszeit von mehreren Stunden durch Filtrieren wieder abgetrennt wird - (US-PS 3 860 656; US-PS 3 507 764).

Der Nachteil der geschilderten diskontinuierlichen Arbeitsweise könnte durch Verwendung von granulierter Aktivkohle, die in Adsorptionstürmen angeordnet ist, vermieden werden. Die Erfahrung zeigt jedoch, daß die spezifische Entfärbungsleistung der Aktivkohle sehr stark von der Korngröße abhängig ist und der Verbrauch an Aktivkohle bei granulierter Ware auf mehr als das 10fache ansteigt.

Es wurde nun gefunden, daß man wäßrige Glyoxallösungen erheblich vorteilhafter dadurch entfärben kann, daß man die wäßrige Glyoxallösung über eine im Festbett angeordnete granulierte Aktivkohle leitet, deren differentielle Porenradienverteilung ein Maximum von 10 bis 100 A aufweist, und wobei die Peclet-Zahl zwischen 500 und 5000 liegt, das Aktivkohlebett nach dem Ablauf der entfärbten Glyoxallösung zur Regenerierung mit einer wäßrigen Lösung eines alkalisch wirkenden Mittels und danach mit einer wäßrigen Säure behandelt, und über die so regenerierte Aktivkohle erneut zu entfärbende Glyoxallösung leitet.

Das neue Verfahren ist zur Entfärbung der z.B. bei der technischen Synthese erhältlichen 10 bis 60 gew.%igen wäßrigen Glyoxallösungen geeignet, die üblicherweise Farbzahlen von 50 bis 200 APHA aufweisen.

Als Aktivkohle wird eine granulierte Aktivkohle mit einer Teilchengröße von z.B. 0,5 bis 2,5 mm, vorzugsweise 0,9 bis 1,3 mm, verwendet, deren differentielle Porenradienverteilung ein Maximum von 10 bis 100, vorzugsweise von 20 bis 50 A aufweist und wobei die Peclet-Zahl zwischen 500 und 5000, vorzugsweise zwischen 1500 und 3000 liegt. Die differentielle Porenradienverteilung ist in der VDI-Richtlinie 3 674 definiert. Die Peclet-Zahl gibt das Verhältnis des Stoffstromes in der Strömung zum Diffusionsstrom an der Aktivkohle an (s. "Kennzahlen der Verfahrenstechnik", Hüttig-Verlag, Heidelberg, 1985, Seite 60).

Man leitet die Glyoxallösung bei Temperaturen bis 60°C über das Aktivkohlebett. Vorzugsweise arbeitet man bei Temperaturen zwischen 10 und 50°C. Die Mindestbehandlungszeit liegt etwa bei einer Stunde. Nach dem Ablauf der entfärbten Glyoxallösung wird die Aktivkohle dadurch regeneriert, daß man sie mit der wäßrigen Lösung eines alkalisch wirkenden Mittels behandelt. Als alkalisch wirkende Mittel verwendet man zweckmäßigerweise starke Alkalien, wie KOH oder NaOH. Bevorzugt nimmt man 0,2 bis 10 %ige Natron-oder Kalilauge. Nach der Behandlung mit der alkalischen Lösung spült man die Aktivkohle mit einer verdünnten wäßrigen Säure. Geeignete Säuren sind z.B. Schwefelsäure, Phosphorsäure, Ameisensäure oder Salpetersäure. Vorzugsweise nimmt man eine 0,5 bis 15 %ige Salpetersäure. Die Regenerierung führt man bei Temperaturen bis zu 90°C, zweckmäßig bei Raumtemperatur durch.

Nach dem erfindungsgemäßen Verfahren lassen sich wäßrige Glyoxallösungen auf besonders vorteilhafte Weisen entfärben. Daß granulierte Aktivkohle mit der bestimmten differentiellen Porenverteilung und bei den angegebenen Peclet-Zahlen die hier gefundene hohe Entfärbungsleistung aufweist und sich auf so einfache Weise unter Rückgewinnung der ursprünglichen Entfärbungskapazität regenerieren läßt, ist sehr überraschend.

Die erfindungsgemäße Entfärbung, die eine kontinuierliche Arbeitsweise ermöglicht, führt man zweckmäßigerweise in einem Adsorptionsturm durch, in dem man die Aktivkohle fest anordnet. Man kann aber auch andere an sich übliche Apparate verwenden, in denen man die Glyoxallösung mit der Aktivkohle in Berührung bringen kann.

Beispiel

Man füllt 1 Liter granulierte Aktivkohle - (Siebfraktion 0,1 bis 0,3 mm) mit einem Maximum in der differentiellen Porenverteilung von 20 A trocken in einen Adsorptionsturm mit 4 cm Durchmesser und einer Länge von 100 cm. Danach wird der Adsorptionsturm mit Wasserstrahlvakuum evakuiert und von unten nach oben langsam mit einer wäßrigen 40 %igen Glyoxal-Lösung mit einer Farbzahl von 57 APHA befüllt.

Die Glyoxallösung wird bei Raumtemperatur mit einer Durchflußgeschwindigkeit von 0,9 m/h kontinuierlich durch den Adsorptionsturm gepumpt. Unter diesen Bedingungen ergibt sich eine Peclet-Zahl von 1500. Bis zu einer Laufzeit von 50 Stunden wird eine Glyoxallösung erhalten, deren Farbzahl auf ≤10 APHA abgesunken ist.

Zum Regenerieren wird der Aktivkohle-Turm nach Ablauf der Glyoxallösung mit Druckluft nachgeblasen und mit Wasser glyoxalfrei gespült. Dann wird das Aktivkohlebett bei Raumtemperatur mit 0,5 gew.%iger Natronlauge bei einer Durchfließgeschwindigkeit von 0,5 m/h von oben nach unten behandelt. Es sind etwa 10-Bettvolumina an verdünnter Natronlauge erforderlich. Die Aktivkohle wird danach mit 1 gew.%iger Salpetersäure gespült, bis im Ablauf ein pH-Wert von kleiner als 4 erreicht ist.

Man füllt nun den Adsorptionsturm erneut mit der zu entfärbenden Glyoxallösung und verfährt wie oben angegeben. Auch bei 10maliger Regenerierung wurde kein Abfall in der Entfärbungsleistung festgestellt.

**Ansprüche**

1. Verfahren zur Entfärbung wäßriger Glyoxallösungen, dadurch gekennzeichnet, daß man die wäßrige Glyoxallösung über eine im Festbett angeordnete granulierte Aktivkohle leitet, deren differentielle Porenradienverteilung ein Maximum von 10 bis 100 A aufweist, und wobei die Peclet-Zahl zwischen 500 und 5000 liegt, das Aktivkohlebett nach dem Ablauf der entfärbten Glyoxallösung zur Regenerierung mit einer wäßrigen Lösung eines alkalisch wirkenden Mittels und danach mit einer wäßrigen Säure behandelt, und über die so regenerierte Aktivkohle erneut zu entfärbende Glyoxallösung leitet.

2. Verfahren nach Anspruch 1, dadurch gekennzeichnet, daß man eine 10 bis 60 gew.%ige wäßrige Glyoxallösung einsetzt.

3. Verfahren nach Anspruch 1, dadurch gekennzeichnet, daß man als wäßrige Lösung eines alkalisch wirkenden Mittels eine 0,2 bis 10 gew.%ige Natron-oder Kalilauge verwendet.

4. Verfahren nach Anspruch 1, dadurch gekennzeichnet, daß man als wäßrige Säure eine 0,5 bis 15 gew.%ige Salpetersäure verwendet.